# EUROPEAN PATENT APPLICATION

(11) **EP 4 177 256 A1**
(43) Date of publication of application: **10.05.2023**
(21) Application number: 20944357.1
(22) Date of filing: 23.10.2020
(51) Int. Cl.: C07D 493/04, C12N 1/20, C12P 17/18, A61K 31/366, A61P 25/28, A23L 33/10, C12R 1/465

(54) **TRICYCLIC DILACTONE COMPOUND, AND PRODUCTION METHOD AND USE THEREOF**

(30) Priority: 06.07.2020 KR 20200083121
(71) Applicant: Molgenbio Co., Ltd., Seoul 08826 (KR)
(72) Inventor: OH, Dong-Chan, Seoul 06337 (KR); KIM, Byung-Yong, Anyang-si, Gyeonggi-do 14017 (KR); SHIN, Jongheon, Seoul 06676 (KR); KIM, Youngsoo, Incheon 21982 (KR); KWON, Yun, Seoul 01367 (KR); SHIN, Jisu, Incheon 21061 (KR)
(74) Representative: Studio Torta S.p.A.
(86) International application number: PCT/KR2020/014554
(87) International publication number: WO 2022/010043

(57) **Abstract**

Provided are a novel tricyclic dilactone compound, a strain producing the same, a method of producing the tricyclic dilactone compound, and use of the tricyclic dilactone compound. The tricyclic dilactone compound has activity of inhibiting aggregation of amyloid-beta and tau proteins, activity of inhibiting apoptosis, and antiinflammation activity, and thus may be used to prevent, treat, or improve various neurodegenerative brain diseases including Alzheimer's disease and cognitive impairment.

## Description

### TECHNICAL FIELD

The present disclosure relates to a novel tricyclic dilactone compound, a strain producing the same, a method of producing the tricyclic dilactone compound, and use of the tricyclic dilactone compound.

### BACKGROUND ART

Alzheimer's disease (AD) is the most common type of neurodegenerative diseases, but drugs that can halt or reverse the pathological progression are yet to be developed. Aggregated amyloid-beta (Aβ) is a key biomarker protein for AD. Monomeric Aβ is pathophysiologically harmless, but the abnormally aggregated form of Aβ has been known to induce apoptosis of neurons, neuroinflammation, and brain atrophy that are closely related to cognitive impairment of an AD patient. As Aβ begins to accumulate in the brain more than 10 years before the onset of symptoms, removing Aβ from the brain has been widely accepted as a mechanism for the AD treatment.

Efforts have been made to develop treatment of the AD, however, the current treatment only inhibits the aggregation of Aβ and tau proteins, thereby slowing down the progression of the AD. In addition, no drug has been approved by the U.S Food and Drug Administration since 'Namenda' (ingredient name: memantine) in 2003. Recently, substances that reduce the formation of amyloids or promote the degradation of amyloid proteins have been developed, but more studies are needed until developed drugs are commercialized through clinical trials (for example, refer Korean Patent Publication Nos. 10-2101258 B1 and 10-2106821 B1).

Accordingly, there is a need to explore novel natural product-derived compounds having excellent therapeutic effects for neurodegenerative diseases including AD.

### DESCRIPTION OF EMBODIMENTS

### TECHNICAL PROBLEM

The present disclosure provides a tricyclic dilactone compound, derivative, stereoisomer, solvate, or pharmaceutically acceptable salt thereof.

The present disclosure provides a bacterium producing a tricyclic dilactone compound, derivative, stereoisomer, solvate, or pharmaceutically acceptable salt thereof.

The present disclosure provides a method of producing a tricyclic dilactone compound,derivative, stereoisomer, solvate, or pharmaceutically acceptable salt thereof.

The present disclosure provides a pharmaceutical composition for preventing or treating neurodegenerative brain disease and a functional health food for preventing or improving neurodegenerative brain disease or cognitive impairment, the pharmaceutical composition and the functional health food each comprising a tricyclic dilactone compound, derivative, stereoisomer, solvate, or pharmaceutically acceptable salt thereof.

The present disclosure provides a method of preventing or treating neurodegenerative brain disease and a method of preventing or improving neurodegenerative brain disease or cognitive impairment by using a tricyclic dilactone compound, stereoisomer, solvate, or pharmaceutically acceptable salt thereof.

### SOLUTION TO PROBLEM

An aspect of the present disclosure provides a compound represented by Formula 1, derivative, stereoisomer, solvate, or pharmaceutically acceptable salt thereof:

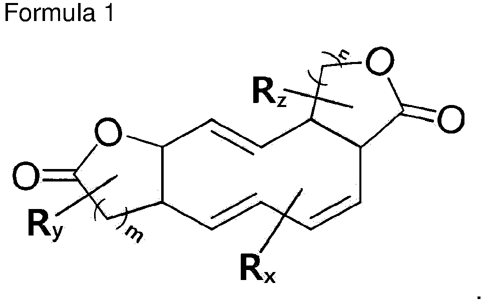

In Formula 1, Rₓ may be one or more substituents in an unsaturated cyclic group. R_{y} may be one or more substituents in a lactone compound. R_{z} may be one or more substituents in a lactone compound.

Rₓ, R_{y}, and R_{z} may each independently be one or more substituents selected from hydrogen, a hydroxy group, a halogen atom, an amine group, a carbonyl group, a cyano group, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, -C(=O)Rₐ, -C(=O)ORₐ, - OCO(ORₐ), -C=N(Rₐ), -SRₐ, -S(=O)Rₐ, -S(=O)₂Rₐ, -PRₐ, a C₂-C₂₀ alkylene oxide group, a substituted or unsubstituted C₆-C₃₀ aryl group, a substituted or unsubstituted C₆-C₃₀ aryloxy group, a substituted or unsubstituted C₆-C₃₀ heteroaryl group, a substituted or unsubstituted C₃-C₃₀ cyclic group, a substituted or unsubstituted C₃-C₂₀ heterocyclic group, or a combination thereof. Here, Rₐ may be hydrogen, a C₁-C₁₀ alkyl group, or a C₆-C₂₀ aryl group.

In Formula 1, m and n may each independently be an integer from 1 to 5. m may be 3. n may be 2.

Here, the compound may be a tricyclic dilactone compound. The term "tricyclic compound" as used herein refers to a compound including three interconnected cyclic groups in a molecule. The term "lactone" as used herein refers to a carboxylic ester compound in a heterocyclic form having a -(C=O)-O- bond in a molecule. The lactone may be γ-butyrolactone, δ-valerolactone, or caprolactone. The term "dilactone" as used herein refers to a compound including two lactone groups in a molecule. The term "tricyclic dilactone" as used herein refers to a compound including three interconnected cyclic groups and two lactone groups in a molecule.

The compound represented by Formula 1 may be represented by Formula 2:

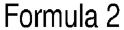

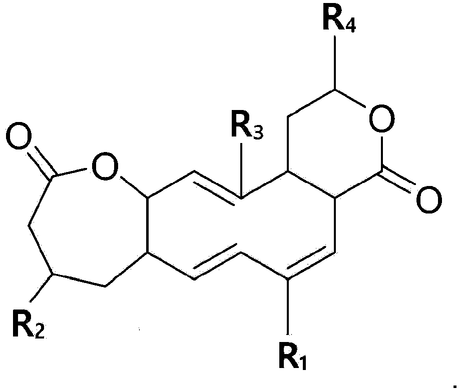

In Formula 2, R₁, R₂, R₃, and R₄ may are each independently be one or more substituents selected from hydrogen, a hydroxy group, a halogen atom, an amine group, a carbonyl group, a cyano group, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, -C(=O)Rₐ, -C(=O)ORₐ, -OCO(ORₐ), -C=N(Rₐ), -SRₐ, -S(=O)Rₐ, -S(=O)₂Rₐ, -PRₐ, a C₂-C₂₀ alkylene oxide group, a substituted or unsubstituted C₆-C₃₀ aryl group, a substituted or unsubstituted C₆-C₃₀ aryloxy group, a substituted or unsubstituted C₆-C₃₀ heteroaryl group, a substituted or unsubstituted C₃-C₃₀ cyclic group, a substituted or unsubstituted C₃-C₂₀ heterocyclic group, or a combination thereof. Here, Rₐ may be hydrogen, a C₁-C₁₀ alkyl group, or a C₆-C₂₀ aryl group.

R₁, R₃, and R₄ may each independently be a substituted or unsubstituted C₁-C₁₀ alkyl group.

R₂ may be hydroxy, a substituted or unsubstituted C₁-C₁₀ alkyl group, a substituted or unsubstituted C₁-C₁₀ alkoxy group, or a substituted or unsubstituted C₆-C₃₀ aryloxy group.

The compound represented by Formula 2 may be represented by Formula 3:

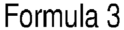

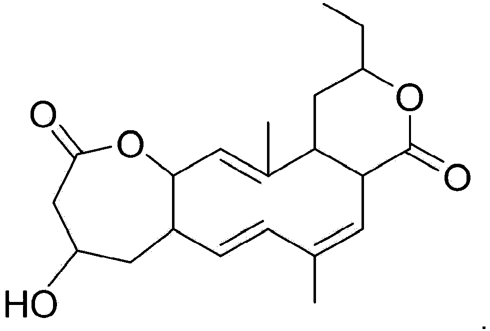

The compound represented by Formula 3 may be represented by Formula 4:

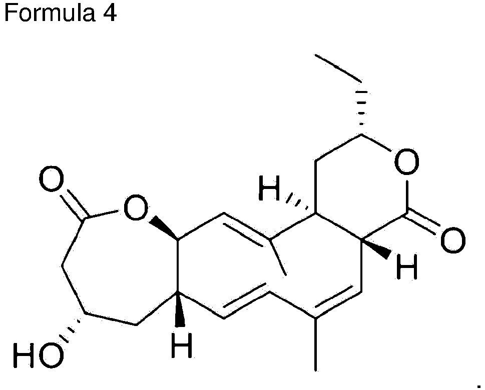

The term "hydroxy" as used herein refers to a -OH functional group (hydroxyl group).

The term "halogen" atom as used herein refers to an atom belonging to Group 17 of the periodic table. The halogen atom includes fluorine, bromine, chlorine, iodine, and the like.

The term "amine" as used herein refers to a functional group in which one or more hydrogen atoms in ammonia (NH₃) are substituted with an alkyl group or an aromatic cyclic group. The amine group may be an amino group (-NH₂).

The term "carbonyl" as used herein refers to a functional group consisting of a carbon atom double-bonded to an oxygen atom, i.e., C=O.

The term "cyano" as used herein refers to a functional group consisting of a carbon atom triple-bonded to a nitrogen atom.

The term "substituted" as used herein with regard to the terms "unsubstituted" or "substituted" refers to introduction of a different atomic group in place of a hydrogen atom when one or more hydrogen atoms in an organic compound are substituted with a different atom group to form a derivative, and the term "substituent" as used herein refers to the introduced atomic group. The substituent may include a halogen atom, a hydroxy group, a nitro group, a cyano group, an amine group, an amidino group, an acetamino group, a hydrazine group, a hydrazone group, a carboxyl group, a sulfonyl group, a sulfamoyl group, a sulfonate group, a phosphate group, a C₁-C₅ alkyl group, a C₁-C₅ alkoxy group, a C₂-C₅ alkenyl group, a C₂-C₅ alkynyl group, a C₃-C₁₀ cycloalkyl group, a C₆-C₁₀ aryl group, a C₆-C₁₀ heteroaryl group, a C₆-C₂₀ arylalkyl group, a C₆-C₂₀ heteroarylalkyl group, or a combination thereof.

The term "alkyl" as used herein refers to a fully saturated, branched or non-branched (or straight or linear) hydrocarbon. The alkyl may be a C₁-C₂₀ alkyl group, a C₁-C₁₅ alkyl group, a C₁-C₁₀ alkyl group, or a C₁-C₅ alkyl group. The alkyl may be, for example, methyl, ethyl, n-propyl, isopropyl, n-butyl, isobutyl, sec-butyl, n-pentyl, isopentyl, neopentyl, iso-amyl, n-hexyl, 3-methylhexyl, 2,2-dimethylpentyl, 2,3-dimethylpentyl, or n-heptyl.

The term "alkoxy" as used herein refers to an alkyl group bonded to an oxygen atom via a single bond. The alkoxy may be, for example, methoxy, ethoxy, or butoxy.

The term "alkenyl" as used herein refers to a branched or non-branched hydrocarbon having one or more carbon-carbon double bonds. The C₂-C₂₀ alkenyl group may be a C₂-C₁₅ alkenyl group, a C₂-C₁₀ alkenyl group, or a C₂-C₅ alkenyl group. The alkenyl group may be, for example, vinyl, aryl, butenyl, isoprophenyl, or isobutenyl.

The term "alkynyl" as used herein refers to a branched or non-branched hydrocarbon having at least one carbon-carbon triple bond. The C₂-C₂₀ alkynyl group may be a C₂-C₁₅ alkynyl group, a C₂-C₁₀ alkynyl group, or a C₂-C₅ alkynyl group. The alkynyl may be, for example, ethynyl, butynyl, isobutynyl, or isopropynyl.

The term "alkylene oxide" as used herein refers to a cyclic group consisting of one oxygen atom and two or more carbon atoms.

The term "aryl" as used herein may be used alone or in combination to refer to an aromatic system including one or more rings, and may also include a group in which an aromatic ring is fused to one or more carbon rings. The C₆-C₃₀ aryl group may be a C₆₋C₁₅ aryl group or a C₆-C₁₀ aryl group. The aryl may be, for example, phenyl, naphthyl, or tetrahydronaphthyl.

The term "aryloxy" as used herein refers to an aryl group bonded to an oxygen atom via a single bond.

The term "heteroaryl" as used herein refers to a monocyclic or bicyclic organic compound in which one or more carbon atoms have been replaced with hetero atoms selected from the group consisting of N, O, P, and S. The heteroaryl group may include one to five hetero atoms and 5 to 10 ring members. Here, S or N may be oxidized to have several oxidation states.

The term "cyclic" or "carbocyclic" group as used herein refers to a saturated or partially unsaturated non-aromatic monocyclic, bicyclic, or tricyclic hydrocarbon group. The cyclic group may include 3 to 30 carbon atoms, 5 to 20 carbon atoms, or for example, 5 to 10 carbon atoms. The monocyclic group may include, for example, cyclopentyl, cyclopentenyl, cyclohexyl, or cyclohexenyl. The bicyclic group may include, for example, bornyl, decahydronaphthyl, bicyclo[2.1.1]hexyl, bicyclo[2.2.1]heptyl, bicyclo[2.2.1]heptenyl, or bicyclo[2.2.2]octylyl. The tricyclic group may include, for example, adamantyl.

The term "heterocyclic" or "heteroring" group as used herein refers to a cyclic hydrocarbon having at least one hetero atom. The heterocyclic group may include 3 to 30 atoms, 3 to 20 atoms, 3 to 10 atoms, 3 to 6, or 3 to 5 atoms. The heterocyclic group may include one or more different hetero atoms in its ring in addition to carbon atoms. The hetero atom may include one or more selected from the group consisting of sulfur, nitrogen, oxygen, and boron. The heterocyclic group may include, for example, an ethylene oxide group or a tetrahydrofuran group.

The term "derivative" as used herein refers to a compound obtained by substituting a part of the compound structure with a different atom or atom group.

The term "isomer" in "stereoisomer" as used herein refers to compounds that have the same molecular formula but differ in the bond connections or spatial arrangement of their atoms. The isomer may include, for example, a structural isomer and a stereoisomer. The stereoisomer may include a diasteromer or an enantiomer. Enantiomers refer to isomers that are non-superimposable mirror images of each other as in the relationship between left and right hands. Enantiomers may be divided into rectus (R, clockwise) and sinister (S, counterclockwise) when four or more substituents are different from each other at the chiral central carbon. Diasteromers refer to stereoisomers that are not mirror images of each other, but are formed by different spatial arrangement of atoms. The diasteromer may be divided into a cistrans isomer and a conformational isomer (also referred to as a conformer).

The term "solvate" as used herein refers to a compound solvated in an organic or inorganic solvent. The solvate may be, for example, a hydrate.

The term "salt" as used herein refers to inorganic and organic acid addition salts of a compound. The pharmaceutically acceptable salt may be a salt that does not cause serious irritation to an organism to which a compound is administered and that does not impair biological activities and properties of the compound. The inorganic acid salt may include hydrochloride, bromate, phosphate, sulphate, or disulfide. The organic salt may include formate, acetate, propionate, lactate, oxalate, tartrate, malate, maleate, citrate, fumarate, besylate, camsylate, edisylate, trichloroacetate, trifluoroacetate, benzoate, gluconate, methanesulfonate, glycolate, succinate, 4-toluenesulfonate, galacturonate, ambonate, glutamate, ethanesulfonate, benzenesulfonate, p-toluenesulfonate, or aspartate. The metal salt may be a calcium salt, a sodium salt, a magnesium salt, a strontium salt, or a potassium salt.

Another aspect of the present disclosure provides a *Streptomyces* sp. WON17 strain deposited under Accession No: KCTC14217BP for producing the compound according to an aspect of the present disclosure, derivative, stereoisomer, solvate, or pharmaceutically acceptable salt thereof.

The strain is capable of producing the compound represented by Formula 1 according to an aspect of the present disclosure, stereoisomer, solvate, or pharmaceutically acceptable salt thereof.

The strain may include a variant thereof. The variant may be, for example, a variant produced by a natural mutation or an artificial mutation. The artificial mutation may be caused by a physical mutagen, such as ultraviolet light, or a chemical mutagen, such as a basic compound.

The strain may include a spore, cell, or culture of the strain.

A further aspect of the present disclosure provides a method of producing the compound represented by Formula 1 according to an aspect of the present disclosure, derivative, stereoisomer, solvate, or pharmaceutically acceptable salt thereof, the method comprising: culturing a *Streptomyces* sp. WON17 strain (Accession No: KCTC14217BP); and
separating the compound represented by Formula 1, derivative, stereoisomer, solvate, or pharmaceutically acceptable salt thereof from the culture.

The *Streptomyces* sp. WON17 strain, the compound represented by Formula 1, the stereoisomer, the solvate, and the pharmaceutically acceptable salt are as described above.

The method may include culturing the *Streptomyces* sp. WON17 strain (Accession No: KCTC14217BP). The culturing may include culturing the strain in a liquid medium or a solid medium. The medium may include a carbon source, such as glucose, starch syrup, dextrin, starch, molasses, animal oil, or vegetable oil. The medium may include a nitrogen source, such as wheat bran, soybean meal, wheat, malt, cottonseed meal, fish meal, corn steep liquor, beef stock, yeast extract, malt extract, ammonium sulfate, sodium nitrate, or urea.

The culturing may be performed while shaking or standing still under aerobic conditions. A temperature for the culturing may be, for example, in a range of about 20°C to about 40°C, about 25°C to about 37°C, or about 28°C to about 35°C, or may be about 30°C. A duration for the culturing may be, for example, in a range of about 1 day to about 4 months, about 1 day to about 2 months, about 1 day to about 6 weeks, about 1 day to about 1 month, about 1 day to about 2 weeks, or about 1 day to about 1 week.

The method may include separating the compound represented by Formula 1, derivative, stereoisomer, solvate, or pharmaceutically acceptable salt thereof from the culture.

The separating may include performing concentration, centrifugation, filtration, or chromatography on the culture. The chromatography may be, for example, column chromatography, planar chromatography, paper chromatography, or thin-film chromatography, depending on a stationary phase type. The chromatography may be, for example, gas chromatography, liquid chromatography, or affinity chromatography, depending on physical properties of a mobile phase. The liquid chromatography may be, for example, high-performance liquid chromatography (HPLC). The chromatography may be, for example, ion-exchange chromatography or sizeexclusion chromatography, depending on the separation method. The chromatography may be, for example, normal-phase chromatography or reversedphase chromatography.

A further aspect of the present disclosure provides a pharmaceutical composition for preventing or treating neurodegenerative brain disease, comprising a compound represented by Formula 1, derivative, stereoisomer, solvate, or pharmaceutically acceptable salt thereof.

The compound represented by Formula 1, the stereoisomer, the solvate, and the pharmaceutically acceptable salt are as described above.

The term "neurodegenerative brain disease" as used herein refers to any disease associated with degenerative changes in the brain, and particularly refers to any (brain) disease that can be caused by one or more factors selected from aggregation of amyloid-beta, aggregation of tau protein, and hyperphosphorylation of tau protein in the brain and/or brain nerve cells.

The neurodegenerative brain disease may be selected from the group consisting of Alzheimer's disease, Parkinson's disease, Huntington's disease, mild cognitive impairment, amyloidosis, multiple system atrophy, multiple sclerosis, tauopathies, Pick's disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia. The amyloidosis may include primary amyloidosis, secondary amyloidosis, hereditary amyloidosis, or focal amyloidosis.

The compound represented by Formula 1 may have activities in inhibiting formation of amyloid-beta aggregates, inhibiting amyloid-beta-induced apoptosis, inhibiting amyloid-beta-induced cell inflammation, and inhibiting formation of tau aggregates.

Human amyloid-beta (Aβ) may be a peptide molecule including about 36 to about 43 amino acids. The Aβ is a major component of amyloid plaques expressed in the brain of a patient with Alzheimer's disease, and is known to be involved in the pathogenesis of Alzheimer's disease. The Aβ may be obtained by cleaving amyloid precursor proteins (APP, UniProtKB P05067) with beta secretase and gamma secretase. The Aβ may be aggregated to form neurotoxic oligomers, thereby causing neurodegenerative brain disease.

The tau protein may consist of four parts, an N-terminal protruding part, a proline aggregation domain, a micro-organelle-binding domain, and a C-terminus (Mandelkow et al., Acta. Neuropathol., 103, 26-35, 1996). The tau protein is known to cause neurodegenerative brain disease, such as Parkinson's disease and tauopathy, when tau is abnormally hyperphosphorylated or altered in neurons of the central nervous system.

The term "prevention" as used herein refers to any action that inhibits a disease or delays the onset of a disease by administering the composition. The term "treatment" as used herein refers to any action that improves or beneficially changes a symptom of a disease by administering the composition.

The pharmaceutical composition may be for administration to a subject in which an aggregation level of the Aβ is higher than normal or at risk of being higher than normal, an aggregation level of the tau protein is higher than normal or at risk of being higher than normal, or a phosphorylation level of the tau protein is higher than normal or at risk of being higher than normal, or a combination thereof. The aggregation level of the Aβ or tau protein may refer to the amount (concentration) of the Aβ aggregates or tau protein aggregates or a ratio of the amount (concentration) of the Aβ aggregates or tau protein aggregates to the total amount (concentration) of Aβ or total amount (concentration) of tau protein. The phosphorylation level of the tau protein may refer to the amount (concentration) of the phosphorylated tau protein or a ratio of the amount (concentration) of the phosphorylated tau protein to the total amount (concentration) of tau protein. The term "normal" as used herein refers to a subject not having the above-defined "neurodegenerative brain disease" among individuals of the same species as a subject (patient) to which the pharmaceutical composition is applied, or brain tissue or cell (brain neurons) separated from the subject and/or cultured.

The pharmaceutical composition comprises the compound represented by Formula 1, derivative, stereoisomer, solvate, or pharmaceutically acceptable salt thereof as an active ingredient. The term "active ingredient" as used herein refers to a physiologically active substance used to achieve pharmacological activity (for example, treatment of neurodegenerative brain disease).

The pharmaceutical composition may further include a known active ingredient having activity in preventing, treating, or improving neurodegenerative brain disease. The known active ingredient having activity in preventing, treating, or improving neurodegenerative brain disease may be an acetylcholinesterase inhibitor or an N-methyl-D-aspartate (NMDA) receptor antagonist. The acetylcholinesterase inhibitor may be tacrine, rivastigmine, galantamine, and donepezil. The NMDA receptor antagonist may be memantine. The compound represented by Formula 1, derivative, stereoisomer, solvate, or pharmaceutically acceptable salt thereof and the known active ingredient may be in a single composition or separate compositions for simultaneous or sequential administration.

The pharmaceutical composition may further include a carrier, an excipient, or a diluent. The carrier, the excipient, and the diluent may include, for example, lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, or mineral oil.

The pharmaceutical composition may be formulated in an oral dosage form, such as powder, granule, tablet, capsule, suspension, emulsion, syrup, aerosol, and the like, an external preparation, a suppository, or a sterile solution for injection, according to respective conventional methods in the art. In the case of formulation, a commonly used diluent or excipient, such as a filler, an extender, a binder, a wetting agent, a disintegrant, a surfactant, and the like, may be used for preparation.

In the pharmaceutical composition, a solid preparation for oral administration may be a tablet, a pill, a powder, a granule, or a capsule. The solid preparation may further include an excipient. The excipient may be, for example, starch, calcium carbonate, sucrose, lactose, or gelatin. In addition, such a solid preparation may further include a lubricant, such as magnesium stearate or talc. In the pharmaceutical composition, a liquid preparation for oral administration may be a suspension, an oral liquid, an emulsion, or a syrup. The liquid preparation may include water or liquid paraffin. The liquid preparation may include an excipient, such as a wetting agent, a sweetening agent, an air freshener, or a preservative. In the pharmaceutical composition, a preparation for parenteral administration may be a sterile aqueous solution, a non-aqueous solution, a suspension, an emulsion, a freeze-dried preparation, or a suppository. The non-aqueous solution or suspension may include vegetable oil or ester. The vegetable oil may be, for example, propylene glycol, polyethylene glycol, or olive oil. The ester may be, for example, ethyl oleate. As a base for the suppository, witepsol, macrogol, tween 61, cacao butter, laurin butter, or glycerogelatin may be used.

The pharmaceutical composition may include an effective amount of the compound represented by Formula 1, derivative, stereoisomer, solvate, or pharmaceutically acceptable salt thereof. The term "effective amount" as used herein refers to an amount sufficient to exhibit an effect of prevention or treatment when administered to a subject in need of prevention or treatment. The effective amount may be appropriately selected by those skilled in the art depending on the cell or subject. A desired dose of the pharmaceutical composition may vary depending on conditions and weight of a subject, severity of a disease, a drug form, and route and duration of administration, but may be appropriately selected by those skilled in the art. However, the compound, derivative, stereoisomer, solvate, or pharmaceutically acceptable salt thereof may be, for example, administered in an amount ranging from about 0.0001 mg/kg to about 100 mg/kg or about 0.001 mg/kg to about 100 mg/kg, 1 to 24 times a day, 1 to 7 times in 2 days to a week, or 1 to 24 times in 1 to 12 months. In the pharmaceutical composition, the compound, derivative, stereoisomer, solvate, or pharmaceutically acceptable salt thereof may be included in an amount ranging from about 0.0001 wt% to about 10 wt% or about 0.001 wt% to about 1 wt%, based on the total weight of the composition.

The administration may be oral or parenteral administration. The administration may be via, for example, oral, transdermal, subcutaneous, rectal, intravenous, intraarterial, intraperitoneal, intramuscular, intrasternal, topical, intranasal, intratracheal, or intradermal route. The composition may be administered systemically or locally, alone or in combination with other pharmaceutically active compounds.

A further aspect of the present disclosure provides a functional health food for preventing or improving neurodegenerative brain disease or cognitive impairment, comprising the compound represented by Formula 1, stereoisomer, solvate, or salt thereof according to an aspect of the present disclosure.

The compound represented by Formula 1, the stereoisomer, the solvate, the salt, the neurodegenerative brain disease, and the prevention are as described above.

The term "cognitive" in "cognitive impairment" as used herein refers to the mental activity or process of gaining knowledge and understanding through thinking, experience, and sensation. Here, processes such as knowledge, attention, memory and working memory, judgment and evaluation, reasoning and computation, problem solving and decision-making, and understanding and generation of language may be included. The cognitive impairment belongs to a category of the mental health impairments that mainly affect learning, memory, perception, and problem solving, and may include amnesia, dementia and delirium.

The term "improvement" as used herein refers to any action that makes a symptom of a disease improved or benefitted.

The functional health food refers to a food prepared by using a raw material or ingredient having a nutrient that is easily deficient in daily meals or having a useful function for the human body (hereinafter, also referred to as 'functional raw material'). The functional health food also refers to a food that helps to maintain health or to prevent and/or improve a certain disease or symptom.

The functional health food may comprise the compound represented by Formula 1, derivative, stereoisomer, solvate, or salt thereof formulated in the form of a capsule, a powder, or a suspension, and may be used as a functional food or added to various foods. The food may include, for example, meat, sausage, bread, chocolate, candy, snack, confectionery, pizza, ramen, other noodles, gum, dairy product including ice cream, various soups, beverage, tea, drinks, alcoholic beverage, vitamin complex, functional food, and health food.

Another aspect of the present disclosure provides a method of preventing or treating neurodegenerative brain disease, the method including administering a pharmaceutical composition for preventing or treating neurodegenerative brain disease to a subject, the pharmaceutical composition including a compound represented by Formula 1, derivative, stereoisomer, solvate, or pharmaceutically acceptable salt thereof according to an aspect of the present disclosure.

The compound represented by Formula 1, the stereoisomer, the solvate, the pharmaceutically acceptable salt thereof, the neurodegenerative brain disease, the prevention, the treatment, and the pharmaceutical composition are as described above.

The subject may include a mammal, such as a human, a mouse, a rat, a cow, a horse, a pig, a dog, a monkey, a sheep, a goat, or a cat. The subject may be suffering from or highly likely to suffer from symptoms associated with neurodegenerative brain disease.

The method may further include administering a known active ingredient for neurodegenerative brain disease to the subject. The known active ingredient may be administered to the subject simultaneously, individually, or sequentially with the compound represented by Formula 1, derivative, stereoisomer, solvate, or pharmaceutically acceptable salt thereof.

The administration may be oral or parenteral administration. The administration may be made via, for example, oral, transdermal, subcutaneous, rectal, intravenous, intraarterial, intraperitoneal, intramuscular, intrasternal, topical, intranasal, intratracheal, or intradermal route. The pharmaceutical composition may be administered systemically or locally, alone or in combination with other pharmaceutically active compounds.

A desired dose of the pharmaceutical composition may vary according to conditions and weight of a patient, severity of disease, a drug form, and route and duration of administration, but may be appropriately selected by those skilled in the art. The dose may be, for example, in a range from about 0.001 mg/kg to about 100 mg/kg, from about 0.01 mg/kg to about 10 mg/kg, or from about 0.1 mg/kg to about 1 mg/kg on an adult basis. The administration may be performed once a day, multiple times a day, once a week, once every 2 weeks, once every 3 weeks, or once every 4 weeks to once a year.

A further aspect of the present disclosure provides a method of preventing or improving neurodegenerative brain disease or cognitive impairment, the method including administering a functional health food to a subject, the functional health food including a compound represented by Formula 1, derivative, stereoisomer, solvate, or salt thereof according to an aspect of the present disclosure.

The compound represented by Formula 1, the stereoisomer, the solvate, the salt, the functional health food, the neurodegenerative brain disease, the prevention, and the improvement are as described above.

### ADVANTAGEOUS EFFECTS OF DISCLOSURE

A tricyclic dilactone compound, a derivative, stereoisomer, solvate, or pharmaceutically acceptable salt thereof have activity of inhibiting aggregation of amyloid-beta and tau protein and inhibiting apoptosis, and anti-inflammatory activity, and thus may be used for prevention, treatment, or improvement of various neurodegenerative brain diseases, such as Alzheimer's disease, and cognitive impairments.

### BRIEF DESCRIPTION OF DRAWINGS

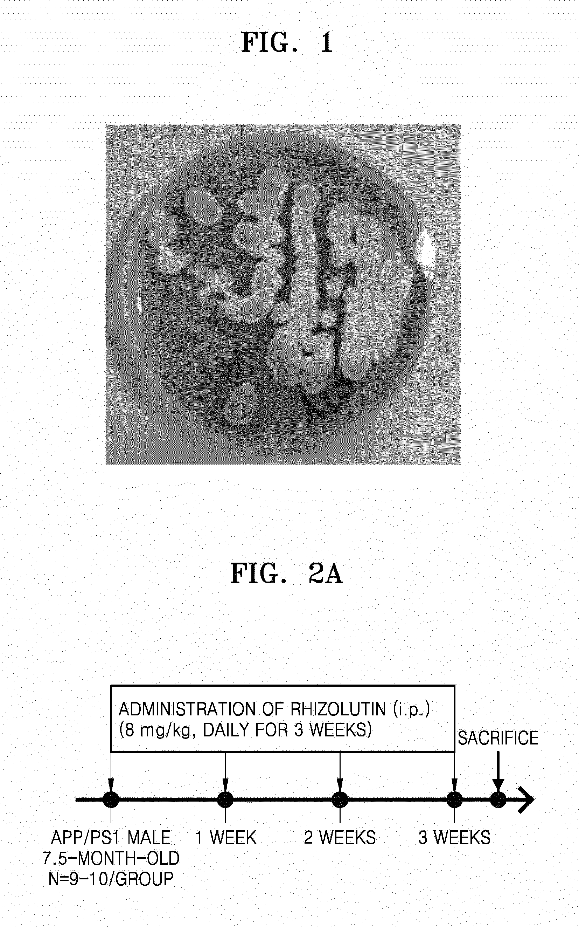

FIG. 1 is a photograph of a medium on which *Streptomyces* sp. WON17 strain was cultured.

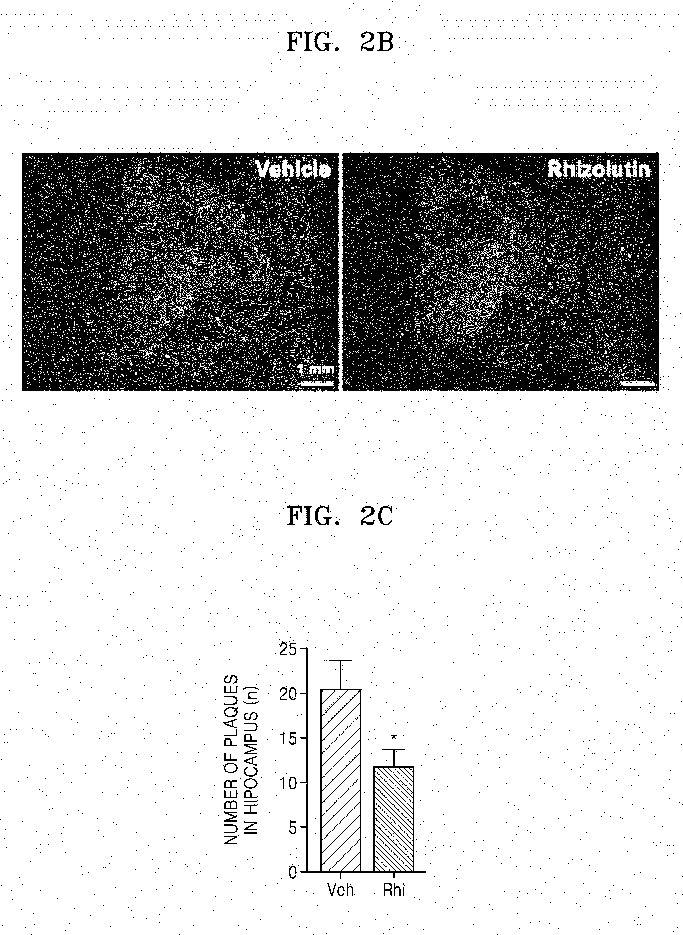

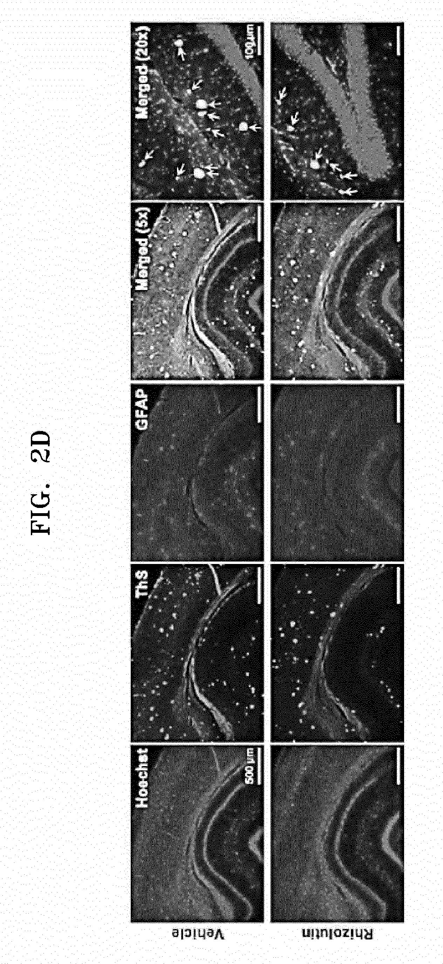

FIG. 2A is a schematic diagram of an experiment to test inhibitory activity of rhizolutin on formation of amyloid-beta aggregates, FIG. 2B shows images of amyloid-beta aggregates stained with thioflavine S in brain tissue, FIG. 2C is a graph showing quantification of plaques in hippocampus (Veh: vehicle, Rhi: rhizolutin, *: p<0.05), and FIG. 2D shows images and merged images of amyloid-beta aggregates stained with GFAP and thioflavine S in brain tissue (ThS: thioflavine S, Merged: merged images).

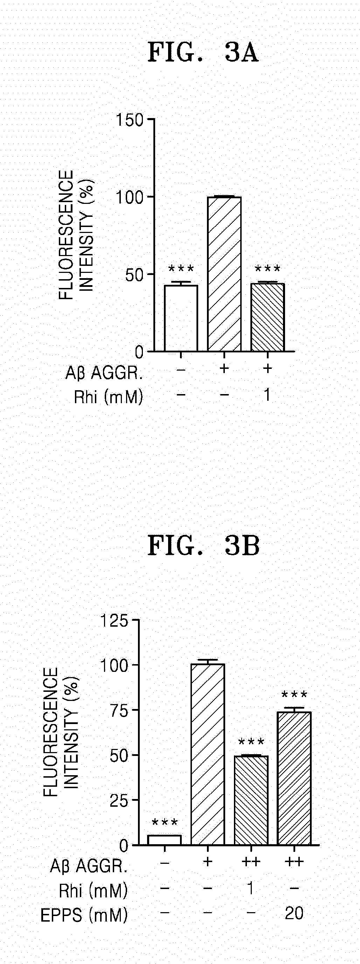

FIG. 3A is a graph showing inhibition of formation of amyloid-beta aggregates in the presence of rhizolutin, and FIG. 3B is a graph showing disaggregation of formed amyloid-beta aggregates in the presence of rhizolutin (y-axis: relative fluorescence intensity (%), Rhi: rhizolutin, *: p<0.05, ***: p<0.001).

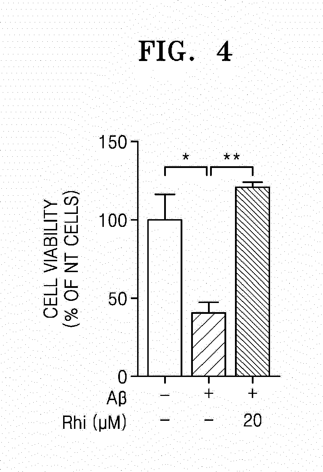

FIG. 4 is a graph showing cell viability (%) of amyloid-beta-treated HT-22 mouse hippocampal neuronal cell line in the presence of rhizolutin (NT: negative control group, Rhi: rhizolutin, *: p<0.05, **: p<0.01).

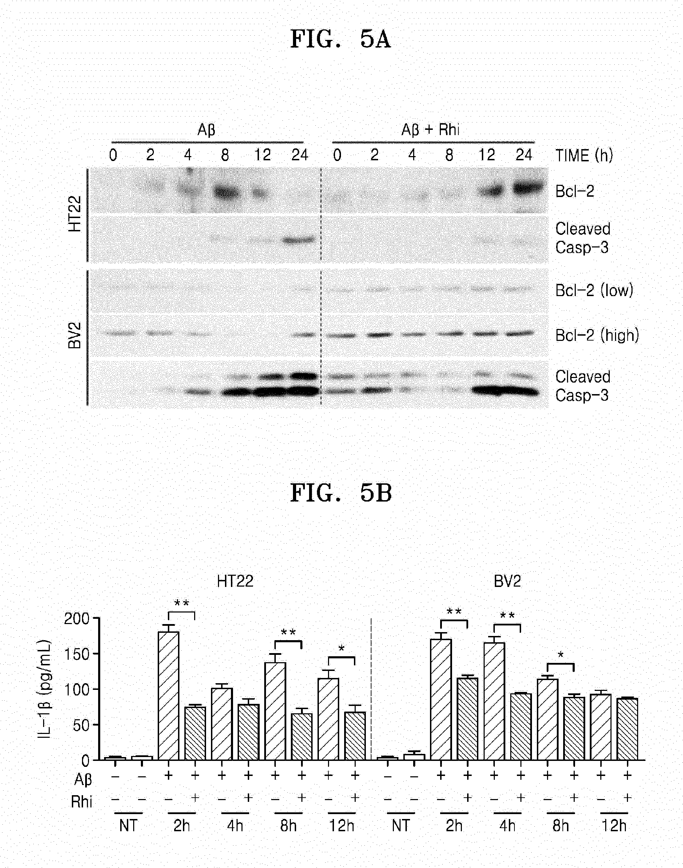

FIG. 5A is an immunoblotting image showing expression of cleaved caspase-3 and Bcl-2 (B-cell lymphoma 2) in HT-22 and BV2 cells treated with amyloid-beta aggregates and rhizolutin, and FIG. 5B is a graph showing amounts of interleukin-1β measured by a sandwich ELISA method (Rhi: rhizolutin, *: p<0.05, **: p<0.01).

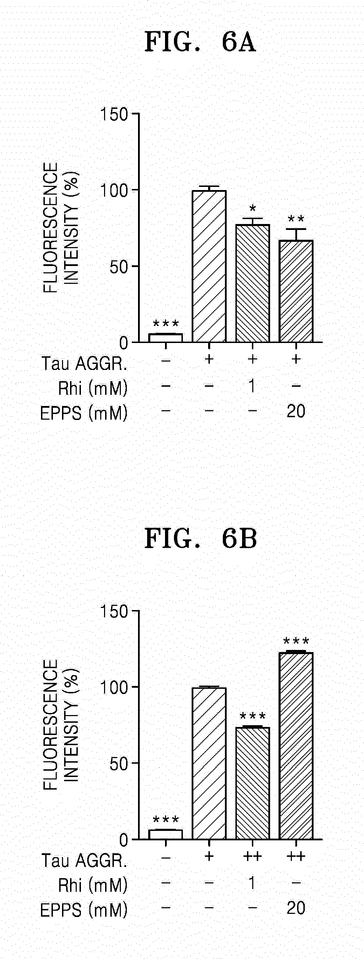

FIG. 6A is a graph showing inhibition of formation of tau protein aggregates in the presence of rhizolutin, and FIG. 6B is a graph showing disaggregation of formed tau protein aggregates in the presence of rhizolutin (y-axis: relative fluorescence intensity (%), Rhi: rhizolutin, **: p<0.01, ***: p<0.001).

### MODE OF DISCLOSURE

Hereinafter, the present disclosure will be described in more detail with reference to Examples below. However, these Examples are for illustrative purposes only, and the scope of the present disclosure is not intended to be limited by these Examples.

### Example 1. Separation of rhizolutin and identification of structure thereof

### 1-1. Isolation of rhizolutin-producing strain

A rhizolutin-producing *Streptomyces* sp. WON17 strain was isolated from the soil for growing *Panax ginseng.* The WON17 strain was isolated from a solid medium (2 g of sodium caseinate, 0.1 g of asparagine, 4 g of sodium propionate, 0.5 g of dipotassium phosphate, 0.1 g of magnesium sulfate, 0.001 g of ferrous sulfate, and 15 g of agar powder per 1 L of sterile water). Based on 16S rDNA sequencing analysis results, the corresponding strain was identified to belong to *Streptomyces* species. The strain was named *Streptomyces* sp. WON17 strain, and was deposited in Korean Collection for Type Culture of Korea Research Institute of Bioscience and Biotechnology on June 17, 2020 under Accession No: KCTC14217BP.

### 1-2. Culture of Streptomyces sp. WON17 strain

The *Streptomyces* sp. WON17 strain was spread on a sterilized YEME solid medium (4 g of yeast extract, 10 g of malt extract, 4 g of glucose, and 18 g of agar per 1 L of distilled water), and cultured at about 30°C for about 4 weeks.

Spores of the cultured WON17 strain were inoculated into a 50 mL-volume YEME liquid medium (4 g of yeast extract, 10 g of malt extract, and 4 g of glucose per 1 L of distilled water), and cultured at 30°C for 5 days while shaking at a rate of 200 rpm. 10 mL of the culture broth was inoculated into a 200 mL-volume modified YEME broth (4 g of yeast extract, 10 g of malt extract, 4 g of glucose, 10 mL of olive oil, and 11 g of dried ginseng powder per 1 L of distilled water), and cultured for about 5 days under conditions of 170 rpm and 30°C.

### 1-3. Separation and purification of rhizolutin

Culture broth of the WON17 strain cultured in Example 1-2 was obtained.

1 L of the WON17 strain culture broth obtained at the end of the culturing and about 1.5 L volume of ethyl acetate (EtOAc, Daejung Chemicals & Metals Co., Ltd.) were added to a separatory funnel mounted on a stand. The funnel was stoppered and shaken in the up, down, right, and left directions for 3 minutes to conduct a first extraction process. Afterwards, the separatory funnel was mounted on a stand again to completely separate water layer and EtOAc layer. The valve of the separatory funnel was then opened to remove the water layer. New EtOAc was added to the water layer, and iterative extractions were performed in the same way. The EtOAc layers were stored separately in a clean flask, and anhydrous sodium sulfate (Daejung Chemicals & Metals Co., Ltd.) was added thereto to remove the water remaining in the culture broth. By passing through several layers of clean gauze, impurities were removed. To remove olive oil, 20 % (v/v) aqueous methanol solution and aqueous hexane solution (at a volume ratio of 1:1) were used. Yield of 99 % was confirmed in the 20 % (v/v) aqueous methanol solution, and the finally treated EtOAc was transferred to a 3 L-volume round flask and dried under reduced pressure using a vacuum drying machine. A total of 700 L of the culture broth was used for extraction to obtain about 210 mg of crude extract.

To purify rhizolutin from the crude extract of the WON17 strain, a purification process was performed thereon using preparative high-performance-liquid-chromatography. After stabilizing a closed column filled with C₁₈ resin with methanol (MeOH) 31 % (v/v)/H₂O 69 % (v/v), separation was conducted under a concentration gradient condition for about 40 minutes until the methanol concentration reached 50 % (v/v). Rhizolutin was detected along with other substances including naphthomycin at about 31 minutes.

To obtain pure rhizolutin from a 30-minute peak fraction dried under reduced pressure, semi-preparative HPLC was conducted under acetonitrile (ACN) 40 % (v/v)/H₂O 60 % (v/v) isocratic conditions. Detailed HPLC conditions are as follows:
(1) A reverse-phase column (C₁₈ (2) Phenomenex Luna, 5 µm 250 mm × 21.2 mm) was used, and separation was performed under a concentration gradient condition of 31 % (v/v) to 50 % (v/v) methanol(MeOH)/aqueous solution(flow rate: 10 mL/min, detection: UV 254 nm). Rhizolutin was identified in a fraction at about 31 minutes.
(2) The fraction obtained at about 31 minutes and dried under reduced pressure was subjected to a reverse-phase column (C₁₈ Phenomenex 250 mm X 4.6 mm), and purification was performed under aq isocratic conditions using 40 % (v/v) ACN/aqueous solution to which 0.1 % (v/v) formic acid was added (flow rate: 1 mL/min, detection: UV 254 nm). Rhizolutin was identified in a fraction at about 28 minutes.

The same process was repeated to obtain about 100 mg of rhizolutin from about 5 g of crude extract.

### 1-4. Identification of chemical structure of rhizolutin

The structure of rhizolutin was identified based on 1D and 2D nuclear magnetic resonance (NMR) spectra. The NMR spectra (¹H NMR, ¹³C NMR) were obtained by using a 500 MHz NMR spectrometer available from Bruker Company, and acetone-*d*₆ as a solvent.

Table 1 shows the structural positioning of rhizolutin by the NMR spectra.

### [Rhizolutin]

(1) Molecular formula: C₂₁H₂₈O₅
(2) Molecular weight: 360
(3) Color: white powder
(4) ¹H-NMR (Pyridine-d₅, 600 MHz): see Table 1
(5) ¹³C-NMR (Pyridine-d₅, 150 MHz): see Table 1

**Table 1**

| Posit ion | Major conformational isomer | | Minor conformational isomer | | 1H HZ Difference |
|---|---|---|---|---|---|
| | δ_{H}, mult (*J* in Hz) | δ_{C} | δ_{H}, mult (*J* in Hz) | δ_{C} | |
| 1 | - | 174.6, s | - | 174.4, s | |
| 2 | 3.69, 1H, t (7.5) | 41.1, d | 3.75, 1H, t (7.5) | 46.8, d | 31.87 |
| 3 | 6.15, 1H, d (7.5) | 125.0, d | 5.92, 1H, d (7.5) | 121.8, d | 139.42 |
| 4 | - | 135.8, s | - | 138.9, s | |
| 5 | 6.12, 1H, m | 139.4, d | 6.14, 1H, m | 138.7, d | 10.78 |
| 6 | 5.30, 1H, dd (16.0, 10.0) | 128.3, d | 5.32, 1H,m | 123.2, d | 12.10 |
| 7 | 3.31, 1H, m | 43.9, d | 3.09, 1H, m | 45.3, d | 128.04 |
| 8 | 1.98, 1H, m | 40.0, t | 1.95, 1H, m | 36.6, t | 18.97 |
| | 2.56, 1H, dt (14.5, 5.0) | | 2.56, 1H, m | | |
| 9 | 4.66, 1H, m | 62.5, d | 4.64, 1H, m | 62.2, d | 15.38 |
| 10 | 3.47, 2H, m | 44.2, t | 3.38, 1H, m | 44.5, t | 58.33 |
| 11 | - | 172.9, s | - | 172.6, s | |
| 12 | 6.13, 1H, m | 83.1, d | 5.88, 1H, m | 80.8, d | 147.46 |
| 13 | 5.04, 1H, d (6.5) | 134.9, d | 5.37, 1H, m | 123.5, d | 195.31 |
| 14 | - | 134.7, q | - | 148.1, t | |
| 15 | 2.63, 1H, t (8.5) | 47.4, s | 2.23, 1H, m | 46.1, d | 237.55 |
| 16 | 1.71, 1H, m | 33.1, t | 1.95, 1H,m | 35.8, d | 145.80 |
| | 1.85, 1H, m | | 2.05, 1H, m | | 119.85 |
| 17 | 4.42, 1H, m | 78.4, d | 4.53, 1H, m | 78.3, d | 67.65 |
| 18 | 1.50, 1H, m | 28.8, t | 1.60, 1H, m | 28.6, t | 55.9 |
| | 1.59, 1H, m | | 1.70, 1H, m | | 63.9 |
| 19 | 0.89, 3H, t (7.5) | 10.0, q | 0.98, 3H, t (7.5) | 10.4, q | 51.61 |
| 20 | 1.76, 3H, s | 13.3, q | 1.74, 3H, s | 24.2, q | 19.31 |
| 21 | 1.92, 3H, s | 20.5, q | 1.91, 3H, s | 20.7, q | 11.30 |

The chemical structural formula of rhizolutin analyzed from the 1D and 2D NMR spectra is shown below:

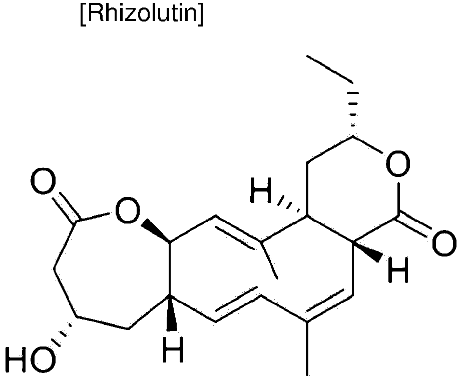

### Example 2. Identification of activity of rhizolutin

### 2-1. Inhibitory activity of rhizolutin on formation of amyloid-beta aggregates

It was to test whether rhizolutin was able to dissolve amyloid-beta aggregates.

First, APP/PS1 transgenic mice (7.5-month-old, male, Jackson Laboratory, 9 to 10 mice/group) to which the genes of AD patients were injected were prepared. In the APP/PS1 transgenic mice, human amyloid-beta is expressed at a high level, and Alzheimer-like plaques are formed in the brain.

The prepared mice were intraperitoneally administered daily with rhizolutin at a dose of 8 mg/body weight (kg)/day for 3 weeks. As a negative control group, the prepared mice were intraperitoneally administered with a vehicle only.

Afterwards, the mouse brain was removed, and changes in amyloid-beta aggregates (in the form of oligomer or fibril) as AD biomarkers in the brain tissue were measured.

FIG. 2B shows images of the amyloid-beta aggregates stained with thioflavine S (available from Sigma-Aldrich) in the brain tissue, and FIG. 2C is a graph showing the quantification of plaques in hippocampus (by unpaired t-test, mean±SEM). As shown in FIG. 2B and FIG. 2C, the group administered with rhizolutin showed decrease in the number of amyloid-beta plaques in the hippocampus of the brain, compared to the negative control group.

FIG. 2D shows images of the amyloid-beta aggregates stained with a glial fibrillary acidic protein (GFAP), which is a biomarker of astrocytosis, and thioflavine S in the brain tissue. As shown in FIG. 2D, it was found that the amount of the amyloid-beta aggregates identified by thioflavine S staining was decreased by the administration of rhizolutin, and that the GFAP, a biomarker of neuroinflammation also decreased to reflect significant reduction in the level of astrocytosis around the plaques.

### 2-2. Activity of inhibiting and disaggregating amyloid-beta aggregation

An amyloid-beta solution was prepared by dissolving human Aβ₁₋₄₂ monomer (UniProt KB-P05067, a.a. 672 to 713) in DMSO to have a concentration of 10 mM. Then, the amyloid-beta solution was diluted with distilled water to prepare a solution having a concentration of 100 µM.

Meanwhile, thioflavine-T (Sigma-Aldrich) was dissolved in 50 mM glycin buffer (pH 8.5) to have a concentration of 5 mM. Then, the resulting solution was diluted with 50 mM glycin buffer (pH 8.5) to have a concentration of 5 µM, and stored light-blocked in a dark room.

To verify the inhibitory activity of rhizolutin on the amyloid-beta aggregation, the amyloid-beta solution was added to 1.5 mL microtubes to a concentration of 25 µM, and rhizolutin was added thereto at a concentration of 1 mM, followed by incubation at 37°C for 120 hours.

25 µl of the reaction solution obtained at the end of reaction was added to each well of a 96-well plate, and 75 µl of the prepared thioflavine-T solution was also added to each well. After a reaction was allowed for 5 minutes at room temperature in the darkroom, fluorescence was measured at an excitation wavelength of 450 nm and an emission wavelength of 485 nm by using a multi-mode microplate reader.

Based on the fluorescence intensity of the group (control group) in which only amyloid-beta was treated and aggregated for 120 hours, the fluorescence intensity (%) of each group was calculated, and results are shown in FIG. 3A (one-way ANOVA test followed by Bonferroni's post-hoc comparison test, mean ± SEM, ***: p<0.001).

In addition, to verify the activity of rhizolutin on disaggregation of the amyloid-beta aggregation, the amyloid-beta solution was added to a 1.5 mL microtube to a concentration of 25 µM and incubated at 37°C for 72 hours. Then, 1 mM of rhizolutin was added to each microtube, followed by incubation at 37°C for 72 hours. To compare the activity, a compound known to effectively disaggregate the amyloid-beta aggregate, i.e., 4-(2-hydroxyethyl)-1-piperazinepropanesulphonic acid (EPPS, CAS Number: 16052-06-5) was incubated at a concentration of 20 mM under the same conditions.

25 *µ*ℓ of the reaction solution obtained at the end of reaction was added to each well of a 96-well plate, and 75 *µ*ℓ of the prepared thioflavine-T solution was added to each well. Then, fluorescence intensity was measured at an excitation wavelength of 450 nm and an emission wavelength of 485 nm by using a multi-mode microplate reader.

Based on the fluorescence intensity of the group (control group) in which only amyloid-beta was treated and aggregated for 72 hours, the fluorescence intensity (%) of each group was calculated, and results are shown in FIG. 3B (mean ± SEM, *: p<0.05, ***: p<0.001).

As shown in FIGS. 3A and 3B, it was confirmed that rhizolutin not only significantly inhibited the formation of the amyloid-beta aggregates (in the form of oligomer or fibril), but also disaggregated the amyloid-beta aggregates (in the form of oligomer or fibril) and the disaggegation activity of rhizolutin was better than that of EPPS, a compound known to have the activity.

### 2-3. Inhibitory activity of rhizolutin on apoptosis

It was to test whether rhizolutin was able to inhibit apoptosis of neurons exposed to neurotoxicity of the amyloid-beta aggregates.

To measure cytotoxicity of rhizolutin, a predetermined number of cells of HT-22 mouse hippocampal neuronal cell line were inoculated into a plate. To the HT-22 cells, an amyloid-beta solution prepared in the same manner as in Example 2-2 was added to a final concentration of 10 µM, followed by incubation at 37°C for 72 hours. Then, 20 µM of rhizolutin was added to each microtube, followed by incubation at 37°C for 24 hours. As a negative control, a vehicle without the amyloid-beta protein and rhizolutin was used.

The viability of the HT-22 cells was measured by MTT(3-(4,5-dimethylthiazol-2-yl)-2,5-diphenyltetrazolium bromide) assay. Based on the viability (%) of the HT-22 cells to which the amyloid-beta was not applied (negative control group), the cell viability (%) of each group was calculated, and results are shown in FIG. 4 (one-way ANOVA, followed by Bonferroni's post-hoc comparison test, mean ± SEM, NT: negative control group, *: p<0.05, **: p<0.01).

As shown in FIG. 4, it was found that the apoptosis of neurons exposed to the neurotoxicity of the amyloid-beta aggregates was inhibited by rhizolutin. Accordingly, rhizolutin was confirmed to have activity of inhibiting apoptosis of neurons caused by the amyloid-beta aggregates.

### 2-4. Verification of inhibitory activity of rhizolutin on apoptosis

To verify the inhibitory activity of rhizolutin on the neurotoxicity of the amyloid-beta aggregates, changes in apoptosis-related biomarkers after exposing neurons to the neurotoxicity of the amyloid-beta aggregates were observed. Here, cleaved caspase-3, which is an apoptosis-inducing factor, and B-cell lymphoma 2 (Bcl-2), which inhibits apoptosis, were used as the biomarkers.

HT-22 mouse hippocampal neuronal cells and BV2 microglial cells were inoculated into a plate in a predetermined number. To each of the HT-22 cell line and the BV2 cell line, an amyloid-beta solution prepared in the same manner as in Example 2-2 was added at a concentration of 1 mM, followed by incubation at 37°C for 24 hours. Afterwards, 20 µM of rhizolutin was added to each microtube, followed by incubation with amyloid-beta in a final concentration of 20 µM at 37°C for 24 hours. As a negative control group, a group without rhizolutin and the amyloid-beta protein was used.

Cleaved caspase-3 and Bcl-2, which are apoptosis-related biomarkers, were identified by immunoblotting, and images thereof are shown in FIG. 5A. As shown in FIG. 5A, due to the amyloid-beta aggregates, the expression of Bcl-2 decreased while the expression of cleaved caspase-3 increased, thereby confirming that the apoptosis of the neurons was induced by the amyloid-beta aggregates. In addition, when treated with the amyloid-beta aggregates and rhizolutin together, the expression of Bcl-2 increased, while the expression of cleaved caspase-3 decreased. Therefore, it was found that the amyloid-beta-induced apoptosis of cells was inhibited by the treatment with rhizolutin.

Meanwhile, in the HT-22 and BV2 cells that were treated with the amyloid-beta aggregates and rhizolutin together, the amount of interleukin-1β (IL-1β), which is a biomarker of inflammation, was measured by sandwitch ELISA method (using unpaired t-test). FIG. 5B is a graph showing the amount of IL-1β depending on the duration of the treatment with the amyloid-beta aggregates and rhizolutin (mean ± SEM, *: p<0.05, **: p<0.01). As shown in FIG. 5B, it was confirmed that, due to the treatment with rhizolutin, the amyloid-beta-induced neuroinflammation of cells was inhibited.

### 2-5. Inhibitory activity of rhizolutin on formation of tau aggregates

It was to test whether rhizolutin was able to inhibit aggregation of tau protein, which is another causative protein of Alzheimer's disease.

Tau protein was prepared at a concentration of 1 mg/mL, and as described in Example 2-3, the prepared tau protein was added to a 1.5 mL microtube at a concentration of 0.5 mg/mL, and 1 mM of rhizolutin and 20 mM of EPPS were added thereto, followed by incubation at 37°C for 120 hours.

Meanwhile, the tau protein was added to a 1.5 mL microtube at a concentration of 0.5 mg/mL, and incubated at 37°C for 48 hours. Then, rhizolutin was added at a concentration of 1 mM, and incubated at 37°C for 72 hours. To compare the efficacy, EPPS was added at a concentration of 20 mM, and incubated under the same conditions.

Thioflavin T was added to the reaction, and the fluorescence intensity was measured at an excitation wavelength of 450 nm and an emission wavelength of 485 nm. As a negative control group, a vehicle without the tau protein and rhizolutin was used.

Based on the fluorescence intensity of the group in which only the tau protein was treated (positive control group), the fluorescence intensity (%) of each group was calculated, and results are shown in FIGS. 6A and 6B (mean ± SEM, *: p<0.01, ***: p<0.001).

As shown in FIGS. 6A and 6B, it was confirmed that, when treated with rhizolutin, the formation of the tau aggregates was inhibited (see FIG. 6A), and at the same time, the existing tau aggregates were disaggregated (see FIG. 6B). When using EPPS, which is known to be effective in the disaggregation of the amyloid-beta aggregates, as a comparison group, it was confirmed that rhizolutin had better effects of inhibiting the formation of the tau aggregates and disaggregating the tau aggregates.

Therefore, it was confirmed that rhizolutin had activities of inhibiting the formation of the amyloid-beta aggregates, inhibiting the amyloid-beta-induced apoptosis, inhibiting the amyloid-beta-induced cell inflammation, and inhibiting the formation of the tau aggregates, and thus would be used to treat Alzheimer's disease.

### [Accession No]

### Name of Depositary Authority: Korea Research Institute of Bioscience and Biotechnology

Accession No : KCTC14217BP

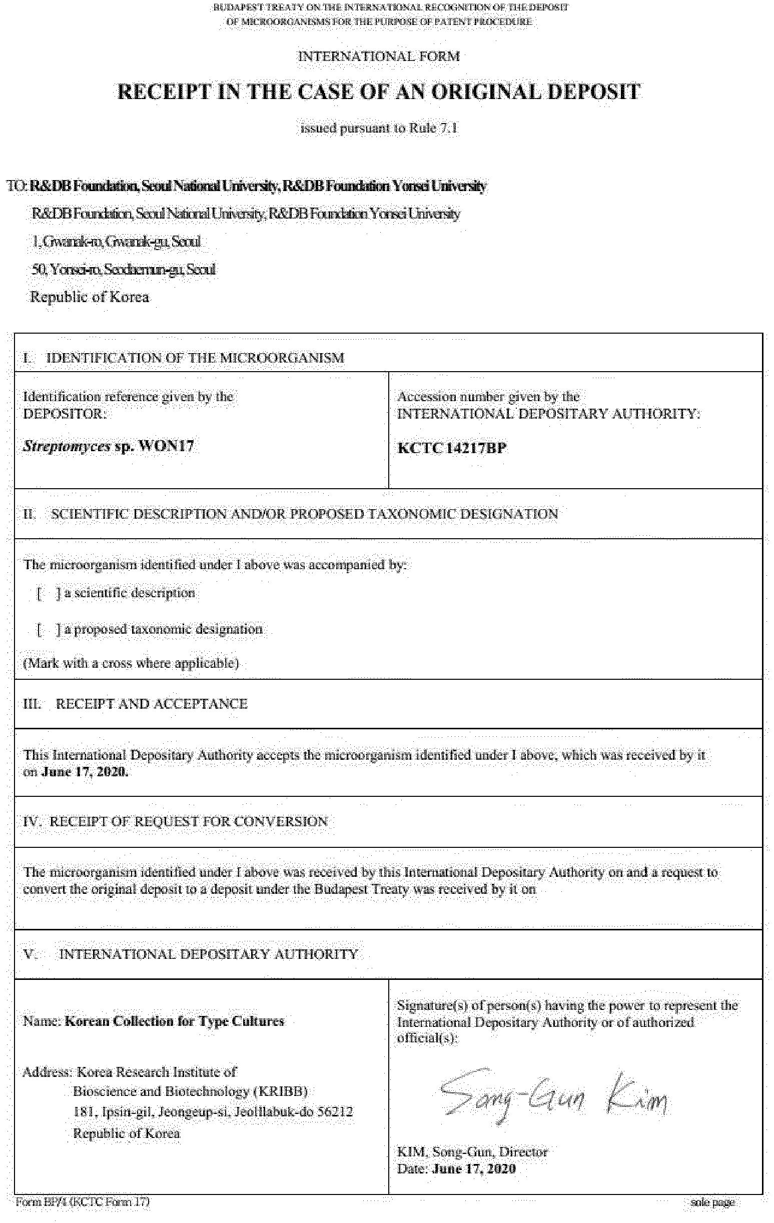
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: R&DB Foundation, Seoul National University, R&DB Foundation Yonsei University

R&DB Foundation, Seoul National University, R&DB Foundation Yonsei University

1, Gwanak-ro, Gwanak-gu, Seoul

50, Yonsei-ro, Seodaemun-gu, Seoul

Republic of Korea

I. IDENTIFICATION OF THE MICROORGANISM
Identification reference given by the
DEPOSITOR:

Streptomyces sp. WON17 | Accession number given by the
INTERNATIONAL DEPOSITARY AUTHORITY:

KCTC 14217BP
II. SCIENTIFIC DESCRIPTION AND/OR PROPOSED TAXONOMIC DESIGNATION
The microorganism identified under I above was accompanied by:

[ ] a scientific description

[ ] a proposed taxonomic designation

(Mark with a cross where applicable)
III. RECEIPT AND ACCEPTANCE
This International Depositary Authority accepts the microorganism identified under I above, which was received by it
on June 17, 2020.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):


KIM, Song-Gun, Director
Date: June 17, 2020

Form BP/4 (KCTC Form 17)    sole page

## Claims

1. A compound represented by Formula 1, derivative, stereoisomer, solvate, or pharmaceutically acceptable salt thereof: 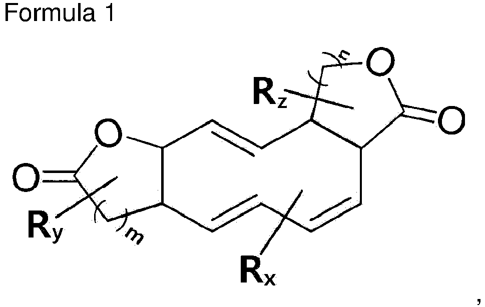
wherein Rₓ, R_{y}, and R_{z} are each independently one or more substituents selected from hydrogen, a hydroxy group, a halogen atom, an amine group, a carbonyl group, a cyano group, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, -C(=O)Rₐ, -C(=O)ORₐ, - OCO(ORₐ), -C=N(Rₐ), -SRₐ, -S(=O)Rₐ, -S(=O)₂Rₐ, -PRₐ, a C₂-C₂₀ alkylene oxide group, a substituted or unsubstituted C₆-C₃₀ aryl group, a substituted or unsubstituted C₆-C₃₀ aryloxy group, a substituted or unsubstituted C₆-C₃₀ heteroaryl group, a substituted or unsubstituted C₃-C₃₀ cyclic group, a substituted or unsubstituted C₃-C₂₀ heterocyclic group, or a combination thereof, wherein Rₐ is hydrogen, a C₁-C₁₀ alkyl group, or a C₆-C₂₀ aryl group, and
m and n are each independently an integer from 1 to 5.

2. The compound, derivative, stereoisomer, solvate, or pharmaceutically acceptable salt thereof of claim 1, wherein the compound represented by Formula 1 is represented by Formula 2: 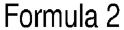 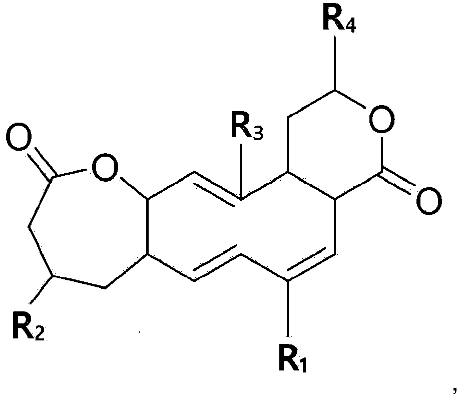 wherein R₁, R₂, R₃, and R₄ are each independently one or more substituents selected from hydrogen, a hydroxy group, a halogen atom, an amine group, a carbonyl group, a cyano group, a substituted or unsubstituted C₁-C₂₀ alkyl group, a substituted or unsubstituted C₁-C₂₀ alkoxy group, a substituted or unsubstituted C₂-C₂₀ alkenyl group, a substituted or unsubstituted C₂-C₂₀ alkynyl group, -C(=O)Rₐ, -C(=O)ORₐ, - OCO(ORₐ), -C=N(Rₐ), -SRₐ, -S(=O)Rₐ, -S(=O)₂Rₐ, -PRₐ, a C₂-C₂₀ alkylene oxide group, a substituted or unsubstituted C₆-C₃₀ aryl group, a substituted or unsubstituted C₆-C₃₀ aryloxy group, a substituted or unsubstituted C₆-C₃₀ heteroaryl group, a substituted or unsubstituted C₃-C₃₀ cyclic group, a substituted or unsubstituted C₃-C₂₀ heterocyclic group, or a combination thereof, wherein Rₐ is hydrogen, a C₁-C₁₀ alkyl group, or a C₆-C₂₀ aryl group.

3. The compound, derivative, stereoisomer, solvate, or pharmaceutically acceptable salt thereof of claim 2, wherein R₁, R₃, and R₄ are each independently a substituted or unsubstituted C₁-C₁₀ alkyl group.

4. The compound, derivative, stereoisomer, solvate, or pharmaceutically acceptable salt thereof of claim 2, wherein R₂ is hydroxy, a substituted or unsubstituted C₁-C₁₀ alkyl group, a substituted or unsubstituted C₁-C₁₀ alkoxy group, or a substituted or unsubstituted C₆-C₃₀ aryloxy group.

5. The compound, derivative, stereoisomer, solvate, or pharmaceutically acceptable salt thereof of claim 2, wherein the compound represented by Formula 2 is represented by Formula 3:

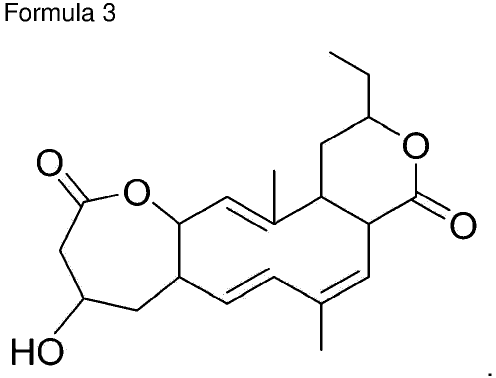

6. The compound, derivative, stereoisomer, solvate, or pharmaceutically acceptable salt thereof of claim 5, wherein the compound represented by Formula 3 is represented by Formula 4:

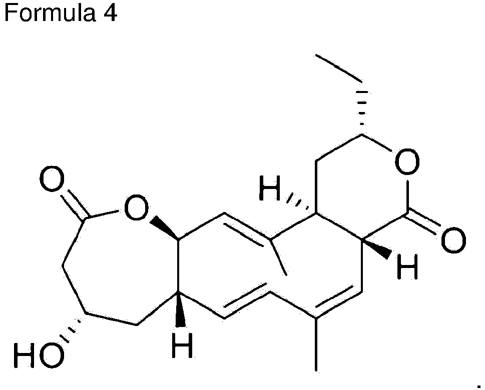

7. A *Streptomyces* sp. WON17 strain deposited under Accession No: KCTC(Korean Collection for Type Cultures)14217BP for producing the compound, derivative, stereoisomer, solvate, or pharmaceutically acceptable salt thereof of claim 1.

8. A method of producing the compound of claim 1, derivative, stereoisomer, solvate, or pharmaceutically acceptable salt thereof, the method comprising:
culturing a *Streptomyces* sp. WON17 strain deposited under Accession No: KCTC14217BP; and
separating the compound of claim 1, derivative, stereoisomer, solvate, or pharmaceutically acceptable salt thereof from the culture.

9. A pharmaceutical composition for preventing or treating neurodegenerative brain disease, the pharmaceutical composition comprising
the compound of claim 1, derivative, stereoisomer, solvate, or pharmaceutically acceptable salt thereof.

10. The pharmaceutical composition of claim 9, wherein the neurodegenerative brain disease is selected from the group consisting of Alzheimer's disease, Parkinson's disease, Huntington's disease, mild cognitive impairment, amyloidosis, multiple system atrophy, multiple sclerosis, tauopathies, Pick's disease, senile dementia, amyotrophic lateral sclerosis, spinocerebellar atrophy, Tourette's syndrome, Friedrich's ataxia, Machado-Joseph's disease, Lewy body dementia, dystonia, progressive supranuclear palsy, and frontotemporal dementia.

11. A functional health food for preventing or improving neurodegenerative brain disease or cognitive impairment, the functional health food comprising
the compound of claim 1, derivative, stereoisomer, solvate, or pharmaceutically acceptable salt thereof.

12. A method of preventing or treating neurodegenerative brain disease, the method comprising
administering a pharmaceutical composition for preventing or treating neurodegenerative brain disease to a subject, the pharmaceutical composition comprising the compound of claim 1, derivative, stereoisomer, solvate, or pharmaceutically acceptable salt thereof.

13. A method of preventing or improving neurodegenerative brain disease or cognitive impairment, the method comprising
administering a functional health food to a subject, the functional health food comprising the compound of claim 1, derivative, stereoisomer, solvate, or pharmaceutically acceptable salt thereof.
